# EUROPÄISCHE PATENTSCHRIFT

(11) **EP 1 210 932 B1**
(45) Veröffentlichungstag und Bekanntmachung des Hinweises auf die Patenterteilung: **03.01.2007**
(21) Anmeldenummer: 01127790.2
(22) Anmeldetag: 22.11.2001
(51) Int. Cl.: A61K 8/81, A61Q 15/00

(54) **Treibgasfreie Sprayzubereitungen**
Propellant free sprays compositions
Compositions de sprays sans gaz propulseur

(30) Priorität: 28.11.2000 DE 10059109
(43) Veröffentlichungstag der Anmeldung: 05.06.2002
(73) Patentinhaber: Henkel Kommanditgesellschaft auf Aktien, 40589 Düsseldorf (DE)
(72) Erfinder: Kemmesies, Andrea, 65185 Wiesbaden (DE); Dickhof, Susanne, 41748 Viersen (DE); Pöppl, Marion, 41564 Kaarst (DE)

(56) Entgegenhaltungen:
- EP-A- 0 599 433
- EP-A- 0 957 119
- EP-A- 0 979 644
- WO-A-98/35651
- FR-A- 2 729 050
- GB-A- 2 113 090
- US-A- 4 534 962

## Beschreibung

Die Erfindung betrifft Sprayprodukte zur topischen Applikation am menschlichen Körper, bestehend aus einem Spendebehälter mit Sprühventil und einer flüssigen, wässrig-alkoholischen Wirkstoffzubereitung, die sich durch günstige Anwendungseigenschaften aufgrund einer besonderen Rheologie auszeichnen.

Für die feine Verteilung von wirkstoffhaltigen Zubereitungen auf dem Körper oder den Haaren haben Aerosolsprays wegen ihrer bequemen Handhabung und der feinen Verteilung, die damit erreicht wird, eine besondere Beliebtheit erlangt. Aus Gründen der ökologischen Bedenklichkeit der Fluorkohlenwasserstoff-Treibgase und der Entflammbarkeit von z. B. Kohlenwasserstoffen und Dimethylether gewinnen aber auch treibgasfreie Spendebehälter mit Sprühventilen zunehmend an Bedeutung. Solche Systeme haben bisher in Form der altbekannten Ballonpumpen für die Parfümzerstäubung, der flexiblen "Squeeze-Bottle" und der starren Sprühflaschen mit Finger- oder Handhebelpumpen praktische Anwendungen gefunden.

Zu den treibgasfreien Spraysystemen zählen darüber hinaus noch die Elastomer-Drucksprühsysteme, in denen ein Elastomer-Beutel in einem starren Gehäuse mit einem Sprühventil in Verbindung steht und mit einem flüssigen Produkt unter Druck abgefüllt ist. Dabei ist die Sprühdruck-Energie in dem Elastomer-Beutel gespeichert.

Die größte Bedeutung haben bisher die Pump-Sprühflaschen mit Finger- oder Handpumpen erlangt.

Aus DE 195 19 404 A1 waren Körperdeodorantien bekannt, die auch aus einem Pump-Spray appliziert werden können. Die Produkte sollen aber weniger als 5 Gew.-% flüchtiger C₁-C₄-Alkohole enthalten. Ein Gehalt an wasserlöslichen Verdickungsmitteln wird darin nur für solche Zubereitungen empfohlen, die zur Anwendung aus Rollkugel-Auftragsbehältem eine Viskosität von 500 - 1000 m·Pa·s (20 °C) aufweisen sollen.

GB-A-2113090 und EP-A-97964 beschreiben treibgasfreie deodoriende Zubereitungen enthaltend Acrylsäure-Homopolymere (Carbomer) als Verdickungsmittel. WO-A-9835651 offenbart die Verwendung von Acrylamid-Polymeren oder Copolymeren in kosmetischen Zubereitungen.

Ein Problem, das bei den meisten aerosolfreien Sprühspendern besteht, ist die Ausbringung eines feinen Sprühstrahls, der die versprühte Flüssigkeit fein zerteilt und in dünner Schicht auf die Körper- oder Hautoberfläche aufbringt, ohne daß die ausgebrachte Flüssigkeit an der Haut oder dem Haar heruntertropft oder auf der Haut ein unangenehmes Nassegefühl erzeugt.

Man hat dieses Ziel durch Entwicklung besonders fein vernebelnder Sprühventile bisher nicht befriedigend gelöst, denn eine extrem feine Zerteilung bzw. Vernebelung ist mit einem erhöhten Kraftaufwand oder einer verringerten Austragsleistung verbunden und kann dazu führen, daß sich das versprühte Produkt nicht am Zielort, z. B. auf der Haut abscheidet, sondern als Nebel in der Luft verbleibt.

Die Erfinder haben sich die Aufgabe gestellt, die Versprühbarkeit von treibgasfreien Sprayzubereitungen und die Abscheidung der versprühten Zubereitungen auf der Haut durch die Formulierung der Zubereitung zu verbessern. Sie haben dabei die Beobachtung gemacht, dass man das Sprühbild und die Abscheidung des Sprays aus einer wässrig-alkoholischen Zubereitung auf der Haut durch die Rheologie der Formulierungen erheblich verbessern kann.

Gegenstand der Erfindung ist eine treibgasfreies Sprayprodukt zur topischen Anwendung am menschlichen Körper, bestehend aus einem Spendebehälter mit Sprühventil und einer flüssigen, wässrig-alkoholischen Wirkstoffzubereitung enthaltend
(A) einen Träger aus Wasser und einem C₂-C₄-Alkohol im Gewichtsverhältnis 1:(0,5-2),
(B) wenigstens einen kosmetischen oder dermatologischen Wirkstoff
(C) 0,01-1 Gew.-% eines im Träger gelösten, polymeren organischen Verdickungsmittels, ausgewählt aus einem Polymer oder Copolymer des Acrylamids, sowie gegebenenfalls bis zu 10 Gew.-% weiterer Hilfsmittel.

Durch den Gehalt des polymeren organischen Verdickungsmittels erhält die Zubereitung ein strukturviskoses Verhalten, d. h. daß die scheinbare Viskosität unter Scherbelastung stark abnimmt. Bei einer Scherbelastung von D = 1/s sollte die scheinbare Viskosität bei 20 °C oberhalb von 100 m·Pa·s, bevorzugt bei 200-500 m·Pa·s liegen, bei einer Scherbelastung von 100/s und mehr sollte die scheinbare Viskosität nicht mehr als 50 % der Viskosität bei 1/s betragen.

Die erfindungsgemäßen Sprayzubereitungen können in jedem beliebigen treibgasfreien Spraysystem, das einen Spendebehälter und ein Sprühventil aufweist, enthalten sein, also z. B. in einer flexiblen Druckflasche mit Tauchrohr und Sprühventil (Squeeze Bottle), in einem Ballonzerstäuber, der nach dem Venturi-Prinzip arbeitet oder in einer Pumpen-Sprühflasche, deren Pumpenhebel mit dem Zeigefinger oder mit der ganzen Hand in der Art eines Abzugsbügels betätigt wird. In einer für die kosmetische Anwendung bevorzugten Ausführung weist der Spendebehälter eine manuell betätigte Sprühpumpe auf.

Das erfindungsgemäße treibgasfreie Sprayprodukt eignet sich zur Verteilung flüssiger Wirkstoffzubereitungen auf festen Oberflächen, bevorzugt auf der Haut oder dem Haar. Als Wirkstoffe können alle bekannten Stoffe mit einer kosmetischen oder dermatologischen Wirkung eingesetzt werden.

Solche Wirkstoffe sind z. B.
- Deodorantien:: z. B. Duftstoffe, antimikrobielle Stoffe, enzymhemmende Stoffe, Antioxydantien oder geruchsadsorbierende Stoffe,
- Antitranspirantien:: z. B. adstringierende, einweißkoagulierende Salze wie Aluminiumchlorhydrat, Aluminium-Zirkoniumtetrachloro-Glycin-Komplex, Zink- oder Aluminium-phenolsulfonat , Zink-glycinat oder Komplexe von basischen Aluminiumchloriden mit Propylenglycol oder Polyethylenglycol

Weitere Wirkstoffe, die mit erfindungsgemäßen, treibgasfreien Sprayzubereitungen angewendet werden können, sind z. B.
- Sonnenschutzmittel (z. B. UV-Filtersubstanzen)
- insektenabweisende Wirkstoffe
- antimikrobielle, fungizide oder desinfizierende Wirkstoffe
- pharmakologische Wirkstoffe, z.B. Sebostatika, Antiphlogistica, Lokalanästhatika, u. a.

Besonders gut eignen sich die erfindungsgemäßen Sprayprodukte zur Applikation von Deodorantien. In einer bevorzugten Ausführung ist daher als Wirkstoff wenigstens ein Deodorant-Wirkstoff, ausgewählt aus antimikrobiellen, enzymhemmenden, antioxydativen Stoffen und Gemischen davon enthalten.

Geeignete antimikrobielle Stoffe sind z. B. halogenierte Phenolderivate wie z. B. Hexachlorophen oder Irgasan DP 300 (Triclosan, 2,4,4'-Trichlor-2'-hydroxydiphenylether), 3,4.4'-Trichlorcarbonilid und Chlorhexidin (1,1'-Hexamethylen-bis-[5-(4-chlorphenyl)]-biguanid). Auch schwächer wirksame antimikrobielle Stoffe, die aber eine spezifische Wirkung gegen die für die Schweißzersetzung verantwortlichen grampositiven Keime haben, können als Deodorant-Wirkstoffe eingesetzt werden. Zu diesen zählen viele ätherische Öle wie z. B. Nelkenöl (Eugenol), Minzöl (Menthol) oder Thymianöl (Thymol) sowie Tarpenalkohole wie z. B. Farnesol. Auch aromatische Alkohole wie z. B. Benzylalkohol, 2-Phenylethanol oder 2-Phenoxyethanol können als Deodorant-Wirkstoffe eingesetzt werden.

Besonders bevorzugt eignen sich langkettige Diole der Formel I

R¹-CHOH-(CHR²)ₓ-CH₂OH (I)

in der x = 0 oder 1 ist und, wenn x = 0 ist, R¹ eine C₆-C₂₂₋Alkylgruppe, eine C₆-C₂₂-Alkoxymethylgruppe, eine 2-Hydroxy (C₆-C₂₂)-alkoxymethylgruppe oder eine C₆-C₂₂-Acyloxymethylgruppe ist und, wenn x = 1 ist, R¹ Wasserstoff und R² eine der für R¹ (x=0) angegebenen Bedeutung hat.

Solche geeigneten Diole sind z.B. 1,2-Dodecandiol, Glycerinmono(C₆-C₁₆)-alkylether oder Glycerinmonolaurat. Diese Verbindungen sind sehr gut haut- und schleimhautverträglich und besitzen eine spezifisch Wirkung gegen Corynebakterien. Als enzymhemmende Stoffe sind vor allem solche deodorierend wirksam, die esterspaltende Lipasen inhibieren und auf diese Weise der Schweißzersetzung entgegenwirken. Hierfür eignen sich vor allem die Ester von aliphatischen C₂-C₆-Carbonsäuren oder Hydroxycarbonsäuren und C₂-C₆-Alkoholen oder Polyolen, z. B. Triethylcitrat, Propylenglycollactat oder Glycerintriacetat (Triacetin).

Antioxidative Stoffe können der oxidativen Zersetzung der Schweißkomponenten entgegenwirken und auf diese Weise die Geruchsentwicklung hemmen. Geeignete Antioxydantien sind z. B. Ascorbylpalmitat, Tocopherole, Tocopherolester und Phenolderivate wie z. B. Butylhydroxytoluol, Butylhydroxyanisol oder Gallussäureester wie Propyl- oder Octylgallat. Auch komplexbildende Stoffe können die deodorierende Wirkung unterstützen, indem sie die oxidativ katalytisch wirkenden Schwermetallionen (z. B. Eisen oder Kupfer) stabil komplexieren.

Geeignete Komplexbildner sind z. B. die Salze der Ethylendiamintetraessigsäure oder der Nitrilotriessigsäure sowie die Salze der 1-Hydroxyethan-1,1-diphosphonsäure. Eine besonders bevorzugte Ausführung der Erfindung ist eine Sprayprodukt zur Verwendung als Deodorant-Spray, bestehend aus einem Spendebehälter mit Sprühventil und manuell betätigter Pumpe und einer wässrig-alkohulischen Wirkstofzubereitung, enthaltend
(A) einen Träger aus Wasser und einen Alkohol, ausgewählt aus Ethanol, Isopropanol und Gemischen davon, in einem Gewichtsverhältnis von 1:(0,5-2)
   (B1) 1-5 Gew.-% eines enzymhemmenden Esters einer aliphatischen C₂-C₆-Carbonsäure oder Hydroxycarbonsäure und eines C₁-C₆-Alkohols oder polyols, der esterspaltende Lipasen inhibiert, und
   (B2) 0,1-1 Gew.-% eines antimikrobiellen Diols der Formel (I)

      R¹-CHOH-(CHR²)ₓ-CH₂OH (I)

      mit spezifischer Wirkung gegen Corynebakterien, in der x=0 oder 1 ist und, wenn x = 0 ist, R¹ eine C₆-C₂₂-Alkylgruppe, eine C₆-C₂₂-Alkoxymethylgruppe, eine 2-Hydroxy-(C₆-C₂₂)-alkoxymethylgruppe oder eine C₆-C₂₂-Acyloxymethylgruppe ist und, wenn x=1 ist, R¹ Wasserstoff und R² eine der für R¹(x=0) angegebenen Bedeutungen hat,
   (B3) 0,01-0,2 Gew.-% Tocopherol oder eines Tocopherolesters
(C) 0,01-1 Gew.-% eines polymeren organischen Verdickungsmittels ausgewählt aus einem Polymer oder Copolymer des Acrylamids, und
   0,1-1 Gew.-% weitere Hilfsmittel, ausgewählt aus Farbstoffen, Duftstoffen, Ölkomponenten, oberflächenaktiven Stoffen und Gemischen davon.

Bevorzugt ist in einer solchen erfindungsgemäßen Deodorant-Sprayzubereitung zusätzlich 0,001 - 0,1 Gew.-% eines im Träger gelösten Lichtschutzmittels enthalten.

Als polymere organische Verdickungsmittel eignen sich alle in den Träger klar löslichen Polymerverbindungen, die der Zubereitung eine gewisse Strukturviskosität verleihen. Hierzu gehören aller wasserlöslichen Polymeren sowie Copolymeren, die in Wasser und wäßrigem Alkohol mit bis zu 65 Gew.-% Alkohol klar löslich sind.

Polymere, die hygroskopisch sind, neigen zum Kleben und sind daher für die Zwecke der vorliegenden Erfindung weniger geeignet. Daher sind Polyvinylpyrrolidon und Polyvinylpyrrolidon-Vinylacetat-Copolymere, wie sie in Haarfestigern üblich sind, für die erfindungsgemäßen Sprayzubereitungen weniger geeignet.

Gut geeignete polymere organische Verdickungsmittel zur Ausführung der vorliegenden Erfindung sind vor allem Polyacrylsäuren und vernetzte Polyacrylsäuren, die z. B. unter der Handelsbezeichnung Carbopol^{®}941, Carbopol^{®}2020, Carbopoh^{®}2050 und Caxbopol^{®} Aqua SF1-Polymer (Goodrich) erhältlich sind. Besonders gut eignen sich vor allem Polyacrylamid und Copolymerisate aus Polyacrylamid und Methacrylsäure oder 2-Acrylamido-2-methylpropansulfonsäure. Solche Polymeren sind zur besseren Einarbeitbarkeit in wässrige Systeme als Emulsionskonzentrat im Handel. Ein für die Zwecke der vorliegenden Erfindung besonders gut geeignetes Verdickungsmittel ist das Handelsprodukt Sepigel^{®} 305. Dabei handelt es sich um eine konzentrierte Emulsion einer Lösung von Polyacrylamid, Laureth-7 und C₁₃-C₁₄-Isoparaffin in Wasser.

Weiter geeignete wasserunlösliche Polymere, die für die Zwecke der vorliegenden Erfindung gut geeignet sind, sind Polyethylenglycole und Polyvinylalkohol (voll verseift). Xanthan-Gum, Methyl-hydroxypropylcellulose und Gemische davon.

Hygroskopische Polymere, die zum Kleben neigen und dabei als Haarfestiger verwendet werden, wie z. B. Polyvinylpyrrolidon und Copolymere aus Vinylpyrrolidon und Vinylacetat, sind für die Zwecke der Erfindung weniger geeignet.

In den erfindungsgemäßen Sprayprodukten ist als polymeres organisches Verdickungsmittel ein Polymer oder Copolymer des Acrylamids enthalten. In einer weiteren, bevorzugten Ausführung ist als polymeres organisches Verdickungsmittel ein Copolymerisat aus Acrylamid und 2-Acrylamido-2-methylpropansulfonsäure in Form eines wasserlöslichen Salzes enthalten. Auch Mischungen der genannten Polymeren können verwendet werden. So ist z. B. als polymeres organisches Verdickungsmittel eine Kombination aus einem Homo- oder Copolymer des Acrylamids und einem vernetzten Homo- oder Copolymer der Acrysäure besonders gut geeignet. Die Einsatzmenge des polymeren Verdickungsmittels richtet sich nach der Art der Polymeren und der übrigen Zusammensetzung des Mittels, sie sollte aber nicht höher sein als zur Ausbildung einer Viskosität von ca. 200-500 m·Pa·s (20 °C) bei einer Scherbelastung von 1/s bzw. von ca. 10-100 m·Pa·s bei einer Scherspannung von 100/s erforderlich ist. In der Regel ist eine Menge von 0,01-1 Gew.-% der polymeren Verdickungsmittel dafür ausreichend.

Neben dem Wirkstoff und dem polymeren organischen Verdickungsmittel können die erfindungsgemäßen Sprayprodukte weitere, für solche Zusammensetzungen übliche Hilfsmittel enthalten. Solche weiteren Hilfsmittel sind z. B.
- Farbstoffe
- Duftstoffe (Parfümöle)
- Ölkomponenten
- Oberflächenaktive Stoffe zur Emulgierung oder Solubilisierung der Duftstoffe, Ölkomponenten oder Wirkstoffe
- Lösungsvermittler
- Säuren, Alkalien oder Puffersalze zur Einstellung oder Stabilisierung des pH-Wertes

Die Menge solcher weiteren Hilfsmittel sollte nicht höher als etwa 10 Gew.-% sein. Der Träger besteht aus einem Gemisch von Wasser und einem C₂-C₄-Alkohol im Gewichtsverhältnis Wasser : Alkohol von 1 : (0,5-2). Als Alkohol wird bevorzugt Ethanol oder Isopropanol oder ein Gemisch dieser Alkohole eingesetzt. In bevorzugten Ausführungen der Erfindung liegt als Gewichtsverhältnis von Wasser : Alkohol bei 1 : (0,7-1,5). Die Wirkstoffe sind bevorzugt im Träger löslich. Wirkstoffe, die im Träger nicht löslich sind, z. B. Öl- oder Wachskomponenten, können auch in emulgierter Form enthalten sein. Dabei ist es bevorzugt, daß die Teilchengröße sehr niedrig ist und in der Größenordnung von Mikroemulsionen (unter 100 Nanometer) oder sogenannten PIT-Emulsionen (unter 500 nm) liegt. Verfahren zur Herstellung von Mikroemulsionen und PIT-Emulsionen sind literaturbekannt.

Die folgenden Beispiele sollen den Erfindungsgegenstand näher erläutern.

### Beispiele

Es wurden Zusammensetzungen gemäß Tabelle I hergestellt. Die Herstellung erfolgte durch Lösen der Bestandteile in Ethanol, zuletzt wurde Wasser und Natronlauge zur Einstellung des pH-Wertes zugesetzt.

**Tabelle I**

| | **1** | **2** | **3** | **4** | **5** |
|---|---|---|---|---|---|
| Ethanol | 40 | 45 | 55 | 55 | 45 |
| Tocopherolacetat | 0,05 | 0,05 | 0,05 | 0,05 | 0,05 |
| Sensiva^{®} SC 50 (1) | 0,1 | 0,1 | 0,1 | 0,1 | 0,1 |
| Triethylcitrat | 3,0 | 3,0 | 3,0 | 3,0 | 3,0 |
| Uvinul^{®} D50 (2) | 0,01 | 0,01 | 0,01 | 0,01 | 0,01 |
| Sepigel^{®} 305 (3) | - | 0,2 | 0,8 | 0,2 | 0,2 |
| Carbopol^{®} 2050 (4) | 0,16 | 0,6 | - | - | - |
| Carbopol Aqua SF-1 (5) | - | - | - | 1,5 | - |
| Carbopol^{®} 2020 (6) | - | - | - | - | 0,04 |
| Eumulgin HRE 40 (7) | 0,5 | 0,5 | 0,5 | 0,5 | 0,5 |
| Parfümöl | 1,2 | 1,2 | 1,2 | 1,2 | 1,2 |
| Wasser + NaOH | ad 100 | ad 100 | ad 100 | ad 100 | ad 100 |
| pH-Wert | 5,83 | 5,70 | 5,74 | 6,51 | 6,09 |
| Viskosität (mPas, 20 °C) Brookfield, Typ RVT m. Helipath | | | | | |
| Spindel 3, 20 U/min | 840 | 150 | 200 | 100 | 200 |

### Anwendungstechnische Prüfung.

Die Zusammensetzungen wurden in durchsichtige Glasflaschen (75 ml) mit Pumpzerstäuber-Aufsatz eingefüllt. Ein Sprühstoß (0,12 g), der waagerecht gegen eine im Abstand von 25 cm stehende vertikale Glasscheibe abgegeben wurde, erzeugte dort einen kreisrunden Flüssigkeitsfilm (ca. 20 cm Ø), von dem keine Tropfen am Glas herablaufen.

Es wurden folgende Handelsprodukte verwendet:

| | | |
|---|---|---|
| (1) | Sensiva^{®} SC 50 | 1-(2-Ethylhexyloxy)-glycerin |
| (2) | Uvinul^{®} D50 | 2,2',4,4'-Tetrahydroxybenzophenon |
| (3) | Sepigel^{®} 305 | INCI: Polyacrylamide, C₁₃-C₁₄-Isoparaffin, Laureth-7 |
| (4) | Carbopol^{®} 2050 | INCI: Carbomer |
| (5) | Carbopol Aqua SF-1 | INCI: Acrylates Copolymer (*früher*: polymer EX 518) |
| (6) | Carbopol^{®} ETD 2020 | INCI: Acrylates / C₁₀-C₃₀-Alkyl Acrylate Cross-Polymer |
| (7) | Eumulgin HRE 40 | hydr. Rizinusöl-Oxethylat (40 EO) |

## Patentansprüche

1. Treibgasfreies Sprayprodukt zur topischen Applikation am menschlichen Körper, bestehend aus einem Spendebehälter mit Sprühventil und einer flüssigen, wässrig-alkoholischen Wirkstoffzubereitung, enthaltend
(A) einen Träger aus Wasser und einem C₂-C₄-Alkohol im Gewichtsverhältnis 1 : (0,5-2),
(B) wenigstens einen kosmetischen oder dermatologischen Wirkstoff
(C) 0,01-1 Gew.-% eines im Träger gelösten, polymeren organischen Verdickungsmittels, ausgewählt aus einem Polymer oder Copolymer des Acrylamids,
sowie gegebenenfalls bis zu 10 Gew.-% weiterer Hilfsmittel.

2. Sprayprodukt gemäß Anspruch 1, **dadurch gekennzeichnet, dass** als polymeres organisches Verdickungsmittel ein Copolymerisat aus Acrylamid und 2-Acrylamido-2-methylpropansulfonsäure in Form eines wasserlöslichen Salzes enthalten ist.

3. Sprayprodukt gemäß einem der Ansprüche 1 oder 2, **dadurch gekennzeichnet, dass** als polymeres organisches Verdickungsmittel eine Kombination aus einem Homo- oder Copolymer des Acrylamids und einem vernetzten Homo- oder Copolymer der Acrylsäure enthalten ist.

4. Sprayprodukt gemäß einem der Ansprüche 1 - 3, **dadurch gekennzeichnet, dass** der Spendebehälter eine manuell betätigte Sprühpumpe aufweist.

5. Sprayprodukt gemäß einem der Ansprüche 1 - 4, **dadurch gekennzeichnet, dass** als Wirkstoff wenigstens ein Deodorant-Wirkstoff, ausgewählt aus antimikrobiellen, enzymhemmenden und antioxidativen Stoffen und Gemischen davon, enthalten ist.

6. Sprayprodukt gemäß einem der Ansprüche 1- 5, **dadurch gekennzeichnet, dass** als weitere Hilfsmittel Ölkomponenten, Emulgatoren, Duftstoffe oder Gemische davon enthalten sind.

7. Sprayprodukt zur Verwendung als Deodorant-Körperspray, bestehend aus einem Spendebehälter mit Sprühventil und manuell betätigter Pumpe und einer wässrig-alkoholischen Wirkstoffzubereitung enthaltend
(A) einen Träger aus Wasser und einem Alkohol ausgewählt aus Ethanol und Isopropanol, in einem Gewichtsverhältnis 1 : (0,5-2)
(B1) 1-5 Gew.-% eines enzymhemmenden Esters einer aliphatischen C₂-C₆-Carbonsäure oder Hydroxycarbonsäure und eines C₁-C₆-Alkohols oder Polyols, der esterspaltende Lipasen inhibiert, und
(B2) 0,1-1 Gew.-% eines antimikrobiellen Diols der Formel (1)
R¹-CHOH-(CHR²)ₓ-CH₂OH (I)
mit spezifischer Wirkung gegen Corynebakterien, in der x = 0 oder 1 ist und, wenn x = 0 ist, R¹ eine- C₆-C₂₂-Alkylgruppe, eine C₆-C₂₂-Alkoxymethylgruppe, eine 2-Hydroxy-(C₆-C₂₂)-alkoxymethylgruppe oder eine C₆-C₂₂-Acyloxymethylgruppe ist und, wenn x=1 ist, R¹ Wasserstoff ist und R¹ eine der für R¹(x=0) angegebenen Bedeutungen hat,
(B3) 0,01-0,2 Gew.-% Tocopherol oder eines Tocopherolesters
(C) 0.01-1 Gew.-% eines polymeren organischen Verdickungsmittels ausgewählt aus einem Polymer oder Copolymer des Acrylamids, und 0,1 - 1 Gew.-% weitere Hilfsmittel ausgewählt aus oberflächenaktiven Stoffen, Ölkomponenten, Duftstoffen oder Gemischen davon.

8. Sprayprodukt gemäß Anspruch 7, **dadurch gekennzeichnet, dass** zusätzlich 0,001-0,1 Gew.-% eines im Träger gelösten Lichtschutzmittels enthalten ist.

9. Sprayprodukt gemäß einem der vorstehenden Ansprüche, **dadurch** gekennzeichet, dass im Träger nicht lösliche Wirkstoffe in emulgierter Form mit Teilchengrößen von PIT-Emulsionen (unter 500 nm) oder Mikroemulsionen (unter 100 nm) vorliegen.

10. Sprayprodukt gemäß einem der vorstehenden Ansprüche, **dadurch gekennzeichnet, dass** der mindestens eine kosmetische oder dermatologische Wirkstoff ausgewählt ist aus
- Sonnenschutzmitteln (z. B. UV-Filtersubstanzen),
- insektenabweisenden Wirkstoffen,
- antimikrobiellen, fungiziden oder desinfizierenden Wirkstoffen,
- pharmakologischen Wirkstoffen.

## Claims

1. Propellant-gas-free spray product for topical application on the human body, consisting of a dispensing container with spray valve and a liquid, aqueous-alcoholic active ingredient preparation comprising
(A) a carrier of water and a C₂-C₄-alcohol in the weight ratio 1:(0.5-2),
(B) at least one cosmetic or dermatological active ingredient,
(C) 0.01-1% by weight of a polymeric organic thickener dissolved in the carrier and chosen from a polymer or copolymer of acrylamide,
and optionally up to 10% by weight of further auxiliaries.

2. Spray product according to Claim 1, **characterized in that** the polymeric organic thickener present is a copolymer of acrylamide and 2-acrylamido-2-methylpropanesulphonic acid in the form of a water-soluble salt.

3. Spray product according to one of Claims 1 or 2, **characterized in that** the polymeric organic thickener present is a combination of a homopolymer or copolymer of acrylamide and a crosslinked homopolymer or copolymer of acrylic acid.

4. Spray product according to one of Claims 1 - 3, **characterized in that** the dispensing container has a manually actuated spray pump.

5. Spray product according to one of Claims 1 - 4, **characterized in that** the active ingredient present is at least one deodorant active ingredient chosen from antimicrobial, enzyme-inhibiting and antioxidative substances and mixtures thereof.

6. Spray product according to one of Claims 1 - 5, **characterized in that** the further auxiliaries present are oil components, emulsifiers, fragrances or mixtures thereof.

7. Spray product for use as deodorant body spray consisting of a dispensing container with spray valve and manually actuated pump and an aqueous-alcoholic active ingredient preparation comprising
(A) a carrier of water and an alcohol, chosen from ethanol and isopropanol, in a weight ratio 1:(0.5-2),
(B1) 1-5% by weight of an enzyme-inhibiting ester of an aliphatic C₂-C₆-carboxylic acid or hydroxycarboxylic acid and a C₁-C₆-alcohol or polyol which inhibits ester-cleaving lipases, and
(B2) 0.1-1% by weight of an antimicrobial diol of the formula (I)
R¹-CHOH- (CHR²)ₓ-CH₂OH (I)
with specific action against corynebacteria, in which x = 0 or 1 and, if x = 0, R¹ is a C₆-C₂₂-alkyl group, a C₆-C₂₂-alkoxymethyl group, a 2-hydroxy-(C₆-C₂₂)alkoxymethyl group or a C₆-C₂₂-acyloxymethyl group, and, if x = 1, R¹ is hydrogen and R² has one of the meanings given for R¹ (x = 0),
(B3) 0.01-0.2% by weight of tocopherol or of a tocopherol ester,
(C) 0.01-1% by weight of a polymeric organic thickener chosen from a polymer or copolymer of acrylamide, and 0.1 - 1% by weight of further auxiliaries chosen from surface-active substances, oil components, fragrances or mixtures thereof.

8. Spray product according to Claim 7, **characterized in that** additionally 0.001-0.1% by weight of a photoprotective agent dissolved in the carrier is present.

9. Spray product according to one of the preceding claims, **characterized in that** active ingredients which are not soluble in the carrier are present in emulsified form with particle sizes of PIT emulsions (below 500 nm) or microemulsions (below 100 nm).

10. Spray product according to one of the preceding claims, **characterized in that** the at least one cosmetic or dermatological active ingredient is chosen from
- sunscreens (e.g. UV filter substances),
- insect-repelling active ingredients,
- antimicrobial, fungicidal or disinfecting active ingredients,
- pharmacological active ingredients.

## Revendications

1. Spray exempt de gaz propulseur destiné à l'application topique sur le corps humain, constitué par un récipient distributeur avec une soupape de pulvérisation et une composition de substance(s) active(s) liquide, aqueuse-alcoolique, contenant
(A) un support constitué par de l'eau et un alcool en C₂-C₄ dans un rapport pondéral de 1 : (0,5-2),
(B) au moins une substance active cosmétique ou dermatologique,
(C) 0,01-1% en poids d'un épaississant organique polymère dissous dans le support, choisi parmi un polymère ou un copolymère de l'acrylamide,
ainsi que le cas échéant jusqu'à 10% en poids d'autres adjuvants.

2. Spray selon la revendication 1, **caractérisé en ce qu'**il contient comme épaississant polymère organique un copolymère d'acrylamide et d'acide 2-acrylamido-2-méthylpropanesulfonique sous forme d'un sel soluble dans l'eau.

3. Spray selon une quelconque des revendications 1 ou 2, **caractérisé en ce qu'**il contient comme épaississant polymère organique une combinaison d'un homopolymère ou d'un copolymère de l'acrylamide et d'un homopolymère ou d'un copolymère réticulé de l'acide acrylique.

4. Spray selon une quelconque des revendications 1 à 3, **caractérisé en ce que** le récipient propulseur présente une pompe de pulvérisation actionnée manuellement.

5. Spray selon les revendications 1 à 4, **caractérisé en ce qu'**il contient comme substance active au moins une substance active déodorante choisie parmi les substances antimicrobiennes, inhibitrices d'enzymes et antioxydantes et les mélanges de celles-ci.

6. Spray selon une quelconque des revendications 1 à 5, **caractérisé en ce qu'**il contient comme autres adjuvants des composants huileux, des émulsifiants, des parfums ou des mélanges de ceux-ci.

7. Spray destiné à l'utilisation comme spray corporel déodorant, constitué par un récipient distributeur avec une soupape de pulvérisation et une pompe actionnée manuellement et une composition de substance(s) active(s) aqueuse-alcoolique, contenant
(A) un support constitué par de l'eau et un alcool choisi parmi l'éthanol et l'isopropanol dans un rapport pondéral de 1 : (0,5-2)
(B1) 1-5% en poids d'un ester inhibiteur d'enzyme d'un acide carboxylique aliphatique en C₂-C₆ ou d'un acide hydroxycarboxylique et d'un alcool en C₁-C₆ ou d'un polyol, qui inhibe les lipases dissociant les esters, et
(B2) 0,1-1% en poids d'un diol antimicrobien de formule (I)
R¹-CHOH-(CHR²)ₓ-CH₂OH (I)
avec une action spécifique contre les corynebactéries, dans laquelle x = 0 ou 1 et, lorsque x = 0, R¹ représente un groupe alkyle en C₆-C₂₂, un groupe alcoxyméthyle en C₆-C₂₂, un groupe 2-hydroxy-(alcoxy en C₆-C₂₂)méthyle ou un groupe (acyle en C₆-C₂₂)oxyméthyle et, lorsque x = 1, R¹ représente hydrogène et R² présente une des significations indiquées pour R¹ (x = 0),
(B3) 0,01-0,2% en poids de tocophérol ou d'un ester de tocophérol,
(C) 0,01-1 % en poids d'un épaississant polymère organique, choisi parmi un polymère ou un copolymère de l'acrylamide et 0,1 -1 % en poids d'autres adjuvants choisis parmi les substances tensioactives, les composants huileux, les parfums ou les mélanges de ceux-ci.

8. Spray selon la revendication 7, **caractérisé en ce qu'**il contient en outre 0,001 - 0,1% en poids d'un agent de protection contre la lumière dissous dans le support.

9. Spray selon une quelconque des revendications précédentes, **caractérisé en ce que** des substances actives insolubles dans le support se trouvent sous une forme émulsionnée avec des grosseurs de particules des émulsions PIT (inférieures à 500 nm) ou des microémulsions (inférieures à 100 nm).

10. Spray selon une quelconque des revendications précédentes, **caractérisé en ce que** ladite au moins une substance active cosmétique ou dermatologique est choisie parmi
- les agents de protection solaire (par exemple les substances de type filtre des UV),
- les substances actives repoussant les insectes,
- les substances actives antimicrobiennes, fongicides ou désinfectantes,
- les substances actives pharmacologiques.
